# EUROPÄISCHE PATENTSCHRIFT

(11) **EP 0 846 669 B1**
(45) Veröffentlichungstag und Bekanntmachung des Hinweises auf die Patenterteilung: **26.05.2004**
(21) Anmeldenummer: 97121668.4
(22) Anmeldetag: 09.12.1997
(51) Int. Cl.: C07B 35/02, C07C 29/20, C07C 209/72, B01J 37/02

(54) **Verfahren zur Hydrierung einer aromatischen Verbindung in Gegenwart eines Trägerkatalysators**
Process for the hydrogenation of aromatic compounds in the presence of supported catalysts
Procédé pour l'hydrogénation de composés aromatiques en présence de catalysateurs supportés

(30) Priorität: 09.12.1996 DE 19651129
(43) Veröffentlichungstag der Anmeldung: 10.06.1998
(73) Patentinhaber: BASF AKTIENGESELLSCHAFT, 67056 Ludwigshafen (DE)
(72) Erfinder: Breitscheidel, Boris, Dr., 67117 Limburgerhof (DE); Rühl, Thomas, Dr., 67227 Frankenthal (DE); Flick, Klemens, Dr., 76863 Herxheim (DE); Henkelmann, Jochem, Dr., 68165 Mannheim (DE); Henne, Andreas, Dr., 67433 Neustadt (DE); Lebkücher, Rolf, Dr., 68165 Mannheim (DE)
(74) Vertreter: Isenbruck, Günter, Dr.

(56) Entgegenhaltungen:
- EP-A- 0 324 190
- EP-A- 0 653 243
- EP-A- 0 814 098
- DD-A- 224 315

## Beschreibung

Die vorliegende Erfindung betrifft ein Verfahren zur Hydrierung einer aromatischen Verbindung, in der mindestens eine Hydroxylgruppe an einen aromatischen Kern gebunden ist, oder einer aromatischen Verbindung, in der mindestens eine Aminogruppe an einen aromatischen Kern gebunden ist, in Gegenwart eines Katalysators, der als katalytisch aktive Komponente mindestens ein Metall der I., VII. oder VIII. Nebengruppe des Periodensystems, aufgebracht auf einem Träger, umfaßt.

In einer Ausführungsform betrifft die vorliegende Erfindung ein Verfahren zur Hydrierung einer aromatischen Verbindung, in der mindestens eine Hydroxylgruppe an einen aromatischen Kern gebunden ist, wobei vorzugsweise zusätzlich zur mindestens einen Hydroxylgruppe mindestens eine, gegebenenfalls substituierte, C₁₋₁₀-Alkylgruppe und/oder mindestens eine C₁₋₁₀-Alkoxygruppe an einen aromatischen Kern gebunden ist. Des weiteren werden Monoalkyl-substituierte Phenole im erfindungsgemäßen Verfahren bevorzugt eingesetzt.

Die ein- oder mehrkernigen aromatischen Verbindungen werden dabei in Gegenwart des hierin beschriebenen Katalysators zu den entsprechenden cycloaliphatischen Verbindungen hydriert, wobei die Hydroxylgruppe erhalten bleibt.

Cycloaliphatische Alkohole, insbesondere Alkylcyclohexanole, sind wichtige Zwischenprodukte für die Herstellung verschiedener Duftstoffe, Arzneimittel und anderer organischer Feinchemikalien. Durch katalytische Hydrierung der entsprechenden aromatischen Vorläufer sind die obigen cycloaliphatischen Alkohole bequem zugänglich.

Das Verfahren, Alkylcyclohexanole durch katalytische Hydrierung der entsprechenden Alkylphenole herzustellen, ist bekannt. Die Hydrierung von Alkylphenolen zu den entsprechenden Alkylcyclohexanolen in Gegenwart von Hydrierungskatalysatoren, insbesondere auf Trägem aufgebrachten Katalysatoren, ist vielfach beschrieben.

Als Katalysatoren werden beispielsweise metallisches Rhodium, Rhodium-Platin-, Rhodium-Ruthenium-Legierungen sowie Ruthenium, Palladium oder Nickel auf Katalysatorträgern verwendet. Als Katalysatorträger werden Kohlenstoff, Bariumcarbonat und insbesondere Aluminiumoxid eingesetzt.

In der PL 137 526 ist die Hydrierung von p-tert.-Butylphenol zu p-tert.-Butylcyclohexanol unter Verwendung eines Nickel-Katalysators beschrieben.

In der DE-A-34 01 343 und der EP 0 141 054 ist ein Verfahren zur Herstellung von 2- und 4-tert.-Butylcyclohexanol aus 2- und 4-tert.-Butylphenol durch katalytische Hydrierung beschrieben. Die Hydrierung wird zweistufig ausgeführt, wobei in der ersten Stufe ein Palladium-Katalysator auf einem Al₂O₃-Träger und in der zweiten Stufe ein Ruthenium-Katalysator auf einem Al₂O₃-Träger verwendet wird. Der Metallgehalt auf dem Träger beträgt dabei 0,1 bis 5 Gew.-%. Die Träger sind nicht weiter spezifiziert. Es wird bei einem Druck von 300 bar unter Produktrückführung gearbeitet, und es werden vorzugsweise die cis-tert.-Butylphenole erhalten, wobei 0,1 bis 0,5 % Nebenprodukte anfallen.

Die US 2 927 127 beschreibt ein Verfahren zur Herstellung von p-tert.-Butylcyclohexanol und Estern davon durch katalytische Hydrierung von p-tert.-Butylphenol. Als Katalysatoren werden 5 % Rhodium auf Kohlenstoff, 5 % Palladium auf Bariumcarbonat und 5 % Ruthenium auf Kohlenstoff verwendet. Bei der Verwendung von Ruthenium auf Kohlenstoff wurde mit einem Druck von 70 bis 120 bar und einer Temperatur von 74 bis 93°C gearbeitet. Als Hydrierungsprodukt wurden 66% cis-Isomer erhalten.

In der DE-A-29 09 663 ist ein Verfahren zur Herstellung von cis-Alkylcyclohexanolen durch katalytische Hydrierung der entsprechenden Alkylphenole beschrieben. Als Katalysator wurde Ruthenium auf einem Al₂O₃-Träger verwendet. Es wurde bei Drücken von 40, 60 oder 80 bar gearbeitet. Als Produkt wurden überwiegend cis-Alkylcyclohexanole erhalten, wobei als Nebenprodukt 0,1 bis 1 % Alkylbenzole anfielen.

In einer weiteren Ausführungsform betrifft die vorliegende Erfindung ein Verfahren zur Hydrierung einer aromatischen Verbindung, in der mindestens eine Aminogruppe an einen aromatischen Kern gebunden ist, wobei vorzugsweise zusätzlich zur mindestens einen Aminogruppe mindestens eine, ggf. substituierte, C₁₋₁₀-Alkylgruppe und/oder mindestens eine C₁₋₁₀-Alkoxygruppe an einen aromatischen Kern gebunden ist. Insbesondere bevorzugt werden Monoallyl-substituierte Amine eingesetzt.

Die ein- oder mehrkernigen aromatischen Verbindungen werden dabei in Gegenwart des hierin beschriebenen Katalysators zu den entsprechenden cycloaliphatischen Verbindungen hydriert, wobei die Aminogruppe erhalten bleibt.

Cycloaliphatische Amine, insbesondere gegebenenfalls substituierte Cyclohexylamine und Dicyclohexylamine, finden Verwendung zur Herstellung von Alterungsschutzmitteln für Kautschuke und Kunststoffe, als Korrosionsschutzmittel sowie als Vorprodukte für Pflanzenschutzmittel und Textilhilfsmittel. Cycloaliphatische Diamine werden zudem bei der Herstellung von Polyamid- und Polyurethanharzen eingesetzt und finden weiterhin Verwendung als Härter für Epoxidharze.

Es ist bekannt, cycloaliphatische Amine durch katalytische Hydrierung der entsprechenden ein- oder mehrkernigen aromatischen Amine herzustellen. Die Hydrierung von aromatischen Aminen zu den entsprechenden cycloaliphatischen Aminen in Gegenwart von Hydrierungskatalysatoren, insbesondere auf Trägern aufgebrachten Katalysatoren, ist vielfach beschrieben.

Als Katalysatoren werden beispielsweise Raney-Kobalt mit basischen Zusätzen (JP 43/3180), Nickel-Katalysatoren (US 4 914 239, DE 80 55 18), Rhodium-Katalysatoren (BE 73 93 76, JP 70 19 901, JP 72 35 424), sowie Palladiumkatalysatoren (US 3 520 928, EP 501 265, EP 53 818, JP 59/196 843) verwendet. In der Mehrzahl werden jedoch rutheniumhaltige Katalysatoren eingesetzt.

Aus der DE 21 32 547 ist ein Verfahren zur Hydrierung ein- oder mehrkerniger aromatischer Diamine zu den entsprechenden cycloaliphatischen Aminen bekannt, das in Gegenwart eines suspendierten Rutheniumkatalysators ausgeführt wird.

In der EP 67 058 ist ein Verfahren zur Herstellung von Cyclohexylamin durch katalytische Hydrierung des entsprechenden aromatischen Amins beschrieben. Als Katalysator wird Rutheniummetall in einer fein verteilten Form auf aktivierten Aluminiumpellets verwendet. Nach vier Rückführungen begann der Katalysator seine Wirksamkeit zu verlieren.

Die EP 324 984 betrifft ein Verfahren zur Herstellung eines Gemisches aus gegebenenfalls substituiertem Cyclohexylamin und gegebenenfalls substituiertem Dicyclohexylamin durch Hydrierung von gegebenenfalls substituiertem Anilin unter Verwendung eines Ruthenium und Palladium auf einem Träger enthaltenden Katalysators, der darüber hinaus eine alkalisch reagierende Alkalimetallverbindung als Modifiziermittel enthält. Ein prinzipiell ähnliches Verfahren ist in der EP 501 265 beschrieben, wobei dort der Katalysator als Modifiziermittel Niobsäure, Tantalsäure oder ein Gemisch beider enthält.

In der US 2 606 925 wird ein Verfahren zur Herstellung einer Aminocyclohexyl-Verbindung durch Hydrieren einer entsprechenden aromatischen Verbindung beschrieben, wobei ein Rutheniumkatalysator, dessen aktive katalytische Komponente ausgewählt wird unter elementarem Ruthenium, Rutheniumoxiden, Rutheniumsalzen, in denen das Ruthenium im Anion oder im Kation vorhanden ist, verwendet wird. Wie die Beispiele dieses Verfahrens zeigen, wird auch dort der Katalysator in einer getrennten Stufe hergestellt und getrocknet und nach längerer Trocknungszeit in das Reaktionsgefäß eingebracht.

Ein weiteres Verfahren zur Herstellung von Cyclohexylamin wird in der US 2 822 392 beschrieben, wobei das Hauptaugenmerk dieser Patentschrift auf die Verwendung eines speziellen Reaktors, in dem das Anilin und der Wasserstoff als Ausgangsprodukte im Gegenstrom miteinander zur Umsetzung gebracht werden, gerichtet ist.

Die US 3 636 108 und US 3 697 449 betreffen die katalytische Hydrierung aromatischer, Stickstoff enthaltender Verbindungen unter Verwendung eines Rutheniumkatalysators, der zusätzlich eine Alkalimetallverbindung als Modifiziermittel umfaßt.

Es hat sich bei vielen der oben beschriebenen Verfahren als nachteilig erwiesen, daß bei diesen Umsetzungen nicht selten größere Mengen an Alkylbenzolen sowie weiteren, nicht identifizierbaren Verbindungen, die bei der Hydrierung als Zersetzungs- bzw. Nebenprodukte gebildet werden, auftraten. Diese Nebenprodukte erschweren die Aufarbeitung und Reinigung des Reaktionsproduktes insbesondere dann, wenn z.B. Alkylcyclohexanole als Duftstoffe bzw. zur Herstellung von Duftstoffen verwendet werden sollen. Ferner nimmt die Aktivität vieler der in den oben beschriebenen Verfahren verwendete Katalysatoren rasch ab, und zwar insbesondere dann, wenn die Hydrierung zur Beschleunigung der Reaktionsgeschwindigkeit bei höheren Reaktionstemperaturen durchgeführt wird.

Die Anmelderin selbst hat in einer Reihe von kürzlich eingereichten Patentanmeldungen, DE 196 04 791.9, DE 195 33 718.2, DE 196 24 484.6, DE 196 24 485.4, DE 196 16 822.8, DE 196 22 705.4, die allesamt Verfahren der hier in Rede stehenden Art unter Verwendung von speziellen Trägerkatalysatoren mit Ruthenium und gegebenenfalls weitern Metallen der I., VII. ider VIII. Nebengruppe des Periodensystems betreffen, eingereicht. Mit Hilfe dieser Verfahren zur Hydrierung von wie oben definierten aromatischen Verbindungen ist es möglich, die entsprechenden hydrierten aromatischen Verbindungen in sehr hoher Ausbeute bzw. in nahezu vollständigem Umsatz bei einem gleichzeitig lediglich minimalen Anteil von Nebenprodukten zu erhalten.

Demgemäß lag der vorliegenden Erfindung die Aufgabe zugrunde, ein weiteres Verfahren zur Hydrierung einer wie eingangs definierten aromatischen Verbindung, mit dem sehr hohe Ausbeuten bzw. nahezu vollständiger Umsatz ereicht werden kann, bereitzustellen.

Weiterhin sollte es. mit diesen Verfahren möglich sein, die gewünschten Produkte mit einem lediglich minimalen Anteil von Nebenprodukten bzw. Zersetzungsprodukten zu erhalten und das Verfahren unter hohen Katalysatorbelastungen und langen Standzeiten mit einer extrem hohen Turn-Over-Zahl durchzuführen.

Die oben definierten Aufgaben werden gelöst durch ein Verfahren zur Hydrierung einer aromatischen Verbindung, in der mindestens eine Hydroxylgruppe an einen aromatischen Kern gebunden ist, oder einer aromatischen Verbindung, in der mindestens eine Aminogruppe an einen aromatischen Kern gebunden ist, in Gegenwart eines Katalysators, der als katalytisch aktive Komponente Ruthenium allein oder zusammen mit mindestens einem Metall mindestens ein Metall der I. , VII. oder VIII. Nebengruppe des Periodensystems, aufgebracht auf einem Träger, umfaßt, dadurch gekennzeichnet, daß der Katalysator erhältlich ist durch
a) Lösen der katalytisch aktiven Komponente oder einer Vorläuferverbindung davon in Wasser oder in einem Lösungsmittel,
b) Versetzen der so erhaltenen Lösung mit einem organischen Polymer, das in der Lage ist, mindestens das Zehnfache seines Eigengewichts an Wasser zu binden, wobei ein gequollenes Polymer erhalten wird
c) anschließendes Vermischen des gequollenen Polymers mit einem Katalysator-Trägermaterial, und
d) Formen der so erhaltenen Masse, Trocknen und Calcinieren,wobei unter einer aromatischen Verbindung eine Verbindung eine Verbindung verstanden wird die eine Einheit der Struktur (I) aufweist: 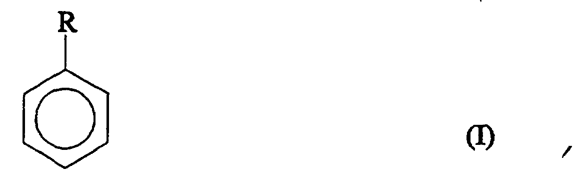 wobei R eine Hydroxyl- oder eine Aminogruppe darstellt.

Die vorstehenden Aufgaben sowie gegebenenfalls weitere Aufgaben werden durch Verfahren zur Hydrierung, wie sie in den Unteransprüchen beschrieben sind, gelöst. Die Besonderheit des erfindungsgemäßen Verfahrens liegt darin, daß sich die aktive Komponente, bedingt durch die oben definierte Herstellungsart, zum überwiegenden Teil in den Makroporen des Trägers befinden, was zu einer ausreichenden Aktivität und hoher Selektivität führt.

Makroporen bezeichnen im Rahmen der vorliegenden Anmeldung Poren, deren Durchmesser oberhalb von 50 nm liegt, und Mesoporen bezeichnen Poren, deren Durchmesser zwischen 2 und 50 nm liegt, wie dies der Definition in *Pure Applied Chem.* 45, S. 79 (1976) entspricht.

Der Begriff *"aromatische Verbindung*, *in der mindestens eine Hydroxylgruppe an einem aromatischen Kern gebunden ist"* bzw. *"aromatische Verbindung, in der mindestens eine Aminogruppe an einen aromatischen Kern gebunden ist"* steht für alle Verbindungen, die eine Einheit der folgenden Struktur (I) aufweisen: 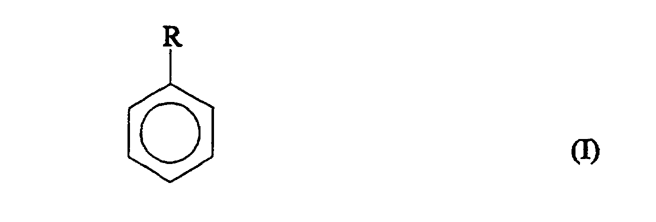 wobei R eine Hydroxyl- oder eine Aminogruppe darstellt.

Sofern im Rahmen des erfindungsgemäßen Verfahrens aromatische Verbindungen eingesetzt werden, in denen mindestens eine Hydroxylgruppe und ferner mindestens ein gegebenenfalls substituierter C₁₋₁₀-Alkylrest und/oder -Alkoxyrest an einen aromatischen Kern gebunden ist, kann je nach Reaktionsbedingungen (Temperatur, Lösungsmittel) das erhaltene Isomerenverhältnis von cis- zu trans-konfigurierten Produkten in einem weiten Bereich variiert werden. Ferner können die erhaltenen Verbindungen ohne weitere Reinigungsschritte weiterverarbeitet werden. Dabei wird die Bildung von Alkylbenzolen praktisch vollständig vermieden.

Wie auch die oben beschriebenen Verbindungen, in denen mindestens eine Hydroxylgruppe an einen aromatischen Kern gebunden ist, können im Rahmen des erfindungsgemäßen Verfahrens auch aromatische Verbindungen, in denen mindestens eine Aminogruppe an einen aromatischen Kern gebunden ist, mit hoher Selektivität zu den entsprechenden cycloaliphatischen Verbindungen hydriert werden, wobei für die zusätzlich mit einem C₁₋₁₀-Alkylrest und/oder -Alkoxyrest substituierten Amine bezüglich des Verhältnisses der cis- und trans-Isomeren das oben Gesagte gilt.

Insbesondere wird im Rahmen dieser Ausführungsform die Bildung von Desaminierungsprodukten, wie beispielsweise Cyclohexanen oder teilhydrierten Dimerisierungsprodukten wie Phenylcyclohexylaminen, praktisch vollständig vermieden.

Ferner weist das erfindungsgemäße Verfahren hohe Turn-Over-Zahlen bei hoher Katalysatorbelastung und über lange Katalysatorstandzeiten hinweg auf. Die Katalysatorbelastung ist dabei die Raum/Zeit-Ausbeute des Verfahrens, d.h. die Menge des umgesetzten Edukts pro Zeiteinheit und pro Menge an vorliegendem Katalysator. Standzeit bedeutet die Zeit bzw. die Menge an umgesetztem Edukt, die ein Katalysator verkraftet, ohne seine Eigenschaften einzubüßen und ohne daß sich die Produkteigenschaften signifikant verändern.

### VERBINDUNGEN

### Aromatische Verbindungen, in denen mindestens eine Hydroxylgruppe an einen aromatischen Kern gebunden ist

Mit dem erfindungsgemäßen Verfahren können aromatische Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, zu den entsprechenden cycloaliphatischen Verbindungen hydriert werden, wobei auch Gemische zweier oder mehrerer dieser Verbindungen eingesetzt werden können. Dabei können die aromatischen Verbindungen einkernige oder mehrkernige aromatische Verbindungen sein. Die aromatischen Verbindungen enthalten mindestens eine Hydroxylgruppe, die an einen aromatischen Kern gebunden ist. Die einfachste Verbindung dieser Gruppe ist Phenol. Vorzugsweise weisen die aromatischen Verbindungen eine Hydroxylgruppe pro aromatischem Kern auf. Die aromatischen Verbindungen können an dem aromatischen Kern oder den aromatischen Kernen substituiert sein durch einen oder mehrere Alkyl- und/oder Alkoxyreste, vorzugsweise C₁₋₁₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Vorzugsweise weisen die erfindungsgemäß hydrierbaren Verbindungen mindestens einen, vorzugsweise einen bis vier, insbesondere einen C₁₋₁₀-Alkylrest auf, der sich vorzugsweise am gleichen aromatischen Kern befindet wie die mindestens eine Hydroxylgruppe. Bevorzugte Verbindungen sind (Mono)alkylphenole, wobei der Alkylrest in o-, m- oder p-Position zur Hydroxylgruppe stehen kann. Insbesondere bevorzugt sind para-Alkylphenole, auch als 4-Alkylphenole bezeichnet, wobei der Alkylrest vorzugsweise 1 bis 10 Kohlenstoffatome aufweist und insbesondere ein tert.-Butylrest ist. Bevorzugt ist 4-tert.-Butylphenol. Erfindungsgemäß verwendbare mehrkernige aromatische Verbindungen sind beispielsweise β-Naphthol und α-Naphthol.

Die aromatischen Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, können auch mehrere aromatische Kerne aufweisen, die über einen Alkylenrest, vorzugsweise eine Methylengruppe verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt sind Methyl-, Ethyl-, Propyl-, Isopropyl-, Butyl- oder tert.-Butylreste, sind.

Dabei kann jeder der aromatischen Kerne mindestens eine Hydroxylgruppe gebunden enthalten. Beispiele solcher Verbindungen sind Bisphenole, die in 4-Position über einen Alkylenrest, vorzugsweise einen Methylenrest, verknüpft sind.

Insbesondere bevorzugt umgesetzt wird im Rahmen des erfindungsgemäßen Verfahrens ein mit einem C₁₋₁₀-Alkylrest, vorzugsweise C₁₋₆-Alkylrest, substituiertes Phenol, wobei der Alkylrest gegebenenfalls mit einem aromatischen Rest substituiert ist, oder Gemische zweier oder mehrerer dieser Verbindungen.

In einer weiteren bevorzugten Ausführungsform dieses Verfahrens wird p-tert.-Butylphenol, Bis(p-hydroxyphenyl)dimethylmethan oder ein Gemisch davon umgesetzt.

### Aromatische Verbindungen, in denen mindestens eine Aminogruppe an einen aromatischen Kern gebunden ist

Mit dem erfindungsgemäßen Verfahren können ferner aromatische Verbindungen, in denen mindestens eine Aminogruppe an einen aromatischen Kern gebunden ist, zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Aminogruppe, die an einen aromatischen Kern gebunden ist. Vorzugsweise sind die aromatischen Verbindungen aromatische Amine oder Diamine. Die aromatischen Verbindungen können an dem aromatischen Kern oder den aromatischen Kernen oder an der Aminogruppe substituiert sein durch einen oder mehrere Alkyl- und/oder Alkoxyreste, vorzugsweise C₁₋₂₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Die aromatische Verbindung, in der mindestens eine Aminogruppe an einen aromatischen Kern gebunden ist, kann auch mehrere aromatische Kerne aufweisen, die über eine Alkylengruppe, vorzugsweise eine Methylengruppe, verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt Methyl-, Ethyl-, Propyl-, Isopropyl-, Butyl-, sec.-Butyl- oder tert.-Butylreste, sind.

Die an den aromatischen Kern gebundene Aminogruppe kann ebenfalls durch einen oder zwei der vorstehend beschriebenen Alkylreste substituiert sein.

Besonders bevorzugte Verbindungen sind Anilin, Naphthylamin, Diaminobenzole, Diaminotoluole, Bis(p-aminophenyl)dimethylmethan und Bis(p-aminophenyl)methan oder Gemische davon.

### KATALYSATOREN

Die erfindungsgemäß verwendeten Katalysatoren sowie deren Herstellung sind an sich bekannt und werden detailliert in der EP-A-0 653 243 beschrieben.

Im folgenden sollen dennoch die prinzipiellen Verfahrensschritte zur Herstellung des erfindungsgemäß verwendeten Katalysators a) bis d) in allgemeiner Weise beschrieben werden.

Die erfindungsgemäß verwendeten Trägerkatalysatoren werden durch die im folgenden beschriebenen Verfahrensschritte hergestellt:

### Verfahrensschritt a):

Die katalytisch aktiven Komponenten oder ihre Vorläuferverbindungen, also Ruthenium alleine oder zusammen mit mindestens einem Metall der I. , VII. oder VIII. Nebengruppe des Periodensystems, vorzugsweise ausgewählt unter Rhodium, Platin, Palladium, Kupfer, Rhenium, Cobalt, Nickel oder ein Gemisch aus zwei oder mehr davon, oder ihre Vorläuferverbindungen, die erst in weiteren Verarbeitungs- oder Aktivierungsschritten in katalytisch aktive Komponenten überführt werden, werden in einem Lösungsmittel gelöst.

Dabei handelt es sich bei dem zu verwendenden Lösungsmittel um Wasser oder polare, mit Wasser mischbare Lösungsmittel, wie z.B. Alkohole, Ether und Amine. Vorzugsweise wird Wasser oder ein Ammoniak-Wasser-Gemisch eingesetzt.

Die katalytisch aktive Komponente wird im allgemeinen in Form eines wasserlöslichen Metallsalzes, wie eines Nitrats, Nitrosylnitrats, Halogenids, als Chlor-, Nitrito- oder Aminkomplex eingesetzt. Derartige Verbindungen sind im Handel erhältlich.

Weiterhin kommen Sole der oben beschriebenen Metalle, wie z.B. Sole der Metalle Palladium, Platin, Silber und Kupfer in Betracht, wie sie z.B. gemäß *Angew. Chem.* 103 (1991) S. 852 oder z.T. im Handel erhältlich sind.

Ferner kann der erfindungsgemäß verwendete Katalysator neben der oben definierten katalytisch aktiven Komponente weiterhin Promotoren oder Moderatoren enthalten, die die katalytische Aktivität oder Selektivität beeinflussen können, und verschieden von dem oder den Aktivmetallen sind. Diese werden direkt oder ebenfalls in Form ihrer Vorläuferverbindungen der Lösung der oben genannten Metallsalze von Ruthenium allein oder zusammen mit mindestens einem Metall der I., VII. oder VIII. Nebengruppe des Periodensystems oder deren Solen zugesetzt.

Die Konzentration von Ruthenium oder zusammen mit mindestens einem Metall der I., VII. oder VIII. Nebengruppe des Periodensystems ist nicht besonders begrenzt und hängt von der Löslichkeit der entsprechenden Verbindung im gewählten Lösungsmittel ab. Sie liegt in der Regel in einem Bereich von mindestens ungefähr 0,1 g/l bis zur Sättigungskonzentration der Lösung. Darüber hinaus richtet sich die Menge der aktiven Komponente nach der im erfindungsgemäß verwendeten Trägerkatalysator verwendeten gewünschten Konzentration. Die oben beschriebenen Lösungen werden im allgemeinen bei Raumtemperatur hergestellt. Weitere Detail zu Verfahrensschritt a) sind der EP-A-O 653234 im Abschnitt *"Verfahrenschritt a) "* zu entnehmen.

### Verfahrensschritt b):

Die oben beschriebenen Lösungen der katalytisch aktiven Komponente oder ihrer Vorläuferverbindungen werden mit einem organischen Polymer versetzt, wobei entweder die Lösung zum Polymer oder das Polymer zur Lösung gegeben werden kann.

Das verwendete organische Polymer ist in der Lage, mindestens das Zehnfache seines Eigengewichts an Wasser zu binden. Solche Verbindungen werden als Hydrogele bezeichnet (vgl. B.D. Rathmer et al. in *"Hydrogels for medical and related applications",* ACS Symposion Series No. 31 (1976)). Es handelt sich hierbei um vernetzte polymere Verbindungen, wobei die Vernetzung durch ionische Wechselwirkungen oder Wasserstoffbrückenbindungen sowie durch chemische Vernetzung erfolgen kann.

Es kommen z.B. Pfropfcopolymerisate aus Stärke und Acrylsäure (z.B. G.F. Fanta et al. in *Starch* 34 (1982), S. 95), Stärke und Acrylsäure (EP-A 83 022), Polysacchariden und Acrylsäure (DE-A 41 05 000), Copolymerisate aus Polyvinylalkohol und Natriumacrylaten (US-A 4,155,893), Copolymerisate aus Acrylamid und Acrylsäure (EP-A 72 214), vernetztes Polyethylenoxid (US-A 3,264,202), vernetztes Polyacrylamid (US-A 3,669,103), vemetztes Poly-N-vinylpyrrolidon (US-A 3,669,103), vernetzte Poylvinylalkohole (Walter et al., *Biomaterials* 9 (1988), S. 150), vernetzte Carboxycellulosefasern (USA 3,826,711), Hydrolysate von Polyvinylacetat-Acrylsäure-Copolymerisaten (GB 20 30 990) und Hydrolysate von Polyacrylnitril (US-A 4,366,206) in Betracht.

Vorzugsweise werden vernetzte Polymere aus Acrylsäure, Acrylsäure und Acrylamid sowie aus Acrylamid verwendet, wobei teilweise neutralisierte Natriumpolyacrylate, die schwach vernetzt sind, besonders bevorzugt verwendet werden, wobei bekannte Vernetzer, wie sie in der EP-A-O 653243 unter *"Verfahrensschritt b)* " beschrieben sind, verwendet werden können.

Im allgemeinen wird das Polymer mit soviel Lösung der aktiven Komponente versetzt, daß es diese vollständig aufnehmen kann. Dieser Vorgang ist im allgemeinen in 60 Minuten beendet; das Quellen des Polymers wird üblicherweise bei Raumtemperatur vorgenommen. Beim Quellen von Polyacrylaten sollte der pH-Wert mindestens 6 betragen, da andernfalls nur eine unzureichende Lösungsaufnahme erfolgt.

### Weitere Details bezüglich Verfahrensschritt b) sind der EP-A-O 653243 unter "Verfahrensschritt b) " zu entnehmen.

### Verfahrensschritt c):

Das gequollene Polymer wird mit einem pulverförmigen Katalysator-Trägermaterial vermischt, wobei es keine Rolle spielt, in welcher Reihenfolge die Komponenten zueinander gegeben werden. Als Trägermaterial kommen unter den Reaktionsbedingungen der zu katalysierenden Reaktion inerte Materialien in Betracht, wobei vorzugsweise Aluminiumoxid, Siliciumdioxid, Kieselgur, ein Kieselgel, eine Tonerde, ein Silicat, ein Zeolith im Gemisch mit einem Aluminiumoxid, ein Zirconiumoxid, ein Titanoxid oder ein Gemisch aus zwei oder mehr davon eingesetzt wird, wobei Aluminiumoxide und Siliciumdioxid besonders bevorzugt sind.

Weiterhin können auch Oxide von Mg, Ca, Sr, Ba, Sulfate von Ca, Ba, Sr, Pb, Carbonate von Mg, Ca, Sr, Ba, Ni, Co, Mn, Fe, Cu, Sulfide von Mo, W, Co, Ni, Fe, Pb, Ag, Cr, Cu, Cd, Sn, Zn, Carbide von B, Si, W und Nitride von B und Si verwendet werden.

Die Menge des Trägermaterials beträgt im allgemeinen das ungefähr 10- bis ungefähr 1000fache, vorzugsweise das ungefähr 20- bis ungefähr 200fache des nicht gequollenen Polymers.

Der Lösung können übliche Peptisierungsmittel zur Verbesserung der mechanischen Stabilität der erhaltenen Formkörper zugesetzt werden, wie z.B. Ammoniak für Aluminiumoxid-Trägermaterialien und Natronlauge für Siliciumdioxid. Die Menge dieser Mittel beträgt in der Regel ungefähr 0,1 bis ungefähr 5 Gew.-%, bezogen auf das Gesamtgewicht des Trägermaterials.

Die oben beschriebenen Komponenten werden vermischt, wozu übliche Kneter oder Mix-Muller benutzt werden können.

### Verfahrensschritt d):

Die nach dem Verfahrensschritt c) erhaltene Masse wird geformt, z.B. durch Extrusion im Extruder oder durch Verformen in einer Strangpresse zu Strängen mit den gewünschten Abmessungen.

Die so erhaltenen Formkörper werden anschließend getrocknet, wobei in der Regel Temperaturen von ungefähr 100°C bis ungefähr 150°C verwendet werden und die Trocknung ungefähr 2 bis ungefähr 24 Stunden lang durchgeführt wird.

Anschließend werden die Formkörper im allgemeinen mehr als 2 bis ungefähr 24 Stunden lang bei ungefähr 300°C bis ungefähr 800°C, vorzugsweise zwischen ungefähr 300°C und 550°C, calciniert. Je nach aktiver Komponente kann sich daran ein Aktivierungsschritt anschließen, wobei die katalytisch aktive Komponente erst gebildet wird.

Die so erhaltenen Trägerkatalysatoren können weiterhin in an sich bekannter Weise auf nicht-poröse Träger aus beispielsweise Steatit, auf Glasringe, Quarzringe oder hochgesinterte Aluminiumoxidringe aufgebracht werden.

Weitere Details bezüglich Verfahrensschritt d) sind der EP-A-O 653241 unter *"Verfahrensschritt d)"* zu entnehmen.

Die Aktivierung der Katalysatoren erfolgt durch Behandlung in einem Gasstrom aus 50 bis 100 Vol.-% H₂ und 0 bis 50 Vol.-% N₂ bei Temperaturen zwischen ungefähr 30°C und ungefähr 600°C, vorzugsweise zwischen ungefähr 150°C und ungefähr 450°C.

Die so erhaltenen Trägerkatalysatoren sind hochporös und weisen ein niedriges Schüttgewicht auf. Durch elektronenmikroskopische Aufnahmen ist deutlich erkennbar, daß sich der überwiegende Teil, in der Regel mehr als 80% der aktiven Komponente, in den Makroporen befindet. Eine Bestimmung des Anteils der Aktivkomponente, der sich in den Makroporen befindet, ist nur durch die Auswertung von mehreren repräsentativen Schnitten durch den Katalysatorstrang möglich, wobei bei der Rasterelektronenmikroskopie die schweren Elemente mit Hilfe der Rückstreuelektronen sichtbar gemacht werden.

Die Reaktionspartner können in den erfindungsgemäßen Katalysatoren die aktiven Zentren leicht erreichen, und die Reaktionsprodukte können leicht abgeführt werden. Durch die Anordnung der katalytisch aktiven Komponenten in den Makroporen ist es möglich, Katalysatoren herzustellen, die, verglichen mit konventionellen Katalysatoren, die gleiche Aktivität aufweisen, aber nur einen Bruchteil der Menge an aktiver Komponente erfordern, wobei vorzugsweise die Menge der katalytisch aktiven Komponente 0,01 bis 30 Gew.-%, weiter bevorzugt 0,05 bis 5 Gew.-% und insbesondere 0,1 bis 3 Gew.-% beträgt.

Der erfindungsgemäß verwendete Katalysator zeichnet sich durch hohe Reaktivität, Selektivität und Standzeit aus.

Nach dem erfindungsgemäßen Verfahren erhält man das entsprechende Hydrierungsprodukt in hoher Ausbeute und Reinheit.

### HYDRIERUNG

Die Hydrierung wird bei geeigneten Drücken und Temperaturen durchgeführt. Bevorzugt sind Drücke oberhalb von ungefähr 5 x 10⁶ Pa, vorzugsweise von ungefähr 1 x 10⁷ bis 3 x 10⁷ Pa. Bevorzugte Temperaturen liegen in einem Bereich von ungefähr 50°C bis ungefähr 300°C, vorzugsweise zwischen ungefähr 100°C und ungefähr 270°C und besonders bevorzugt zwischen ungefähr 150°C und ungefähr 220°C.

Das Hydrierungsverfahren kann kontinuierlich oder in Art eines Batch-Verfahrens durchgeführt werden, und zwar sowohl in Riesel- als auch in Sumpffahrweise. Beim kontinuierlichen Verfahren kann dabei ein Teil des den Reaktor verlassenden Hydrierungsproduktes dem Reaktorzulauf vor dem Reaktor zugeführt werden. Dabei wird eine solche Menge des den Reaktor verlassenden Hydrierungsprodukt als Lösungsmittel rückgeführt, daß die im Unterabschnitt "Lösungs- und Verdünnungsmittel" genannten Mengenverhältnisse erreicht werden. Die verbleibende Menge an Hydrierungsprodukt wird abgezogen.

Bei kontinuierlicher Prozeßführung beträgt die Menge der zur Hydrierung vorgesehenen Verbindung bzw. Verbindungen vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Je nach Reaktionsbedingungen kann bei den zusätzlich durch mindestens einen ggf. substituierten C₁₋₁₀-Alkyl- und/oder -Alkoxyrest substituierten Phenolen und Aminen je nach Reaktionsbedingungen (Temperatur, Lösungsmittel) das erhaltene Isomerenverhältnis von cis- zu trans-konfigurierten Produkten in einem weiten Bereich variiert werden.

Sofern eine aromatische Verbindung, in der mindestens eine Aminogruppe an einen aromatischen Kern gebunden ist, mittels des erfindungsgemäßen Katalysators hydriert werden soll, kann die Hydrierung auch in Gegenwart von Ammoniak oder Dialkylaminen, beispielsweise Methylamin, Ethylamin, Propylamin oder Dimethylamin, Diethylamin oder Dipropylamin durchgeführt werden. Dabei werden geeignete Mengen an Ammoniak oder Mono- oder Dialkylamin eingesetzt, die vorzugsweise bei ungefähr 0,5 bis ungefähr 50 Gewichtsteilen, besonders bevorzugt bei ungefähr 1 bis ungefähr 20 Gewichtsteilen, jeweils bezogen auf 100 Gewichtsteile der zur Hydrierung vorgesehenen Verbindung oder Verbindungen, eingesetzt. Besonders bevorzugt werden wasserfreier Ammoniak oder wasserfreie Amine eingesetzt.

### LÖSUNGS- ODER VERDÜNNUNGSMITTEL

Beim erfindungsgemäßen Verfahren kann die Hydrierung unter Abwesenheit eines Lösungs- oder Verdünnungsmittels durchgeführt werden, d.h. es ist nicht erforderlich, die Hydrierung in Lösung durchzuführen.

Sofern ein Lösungs- oder Verdünnungsmittel eingesetzt wird, kann jedes geeignete Lösungs- oder Verdünnungsmittel eingesetzt werden. Die Auswahl ist dabei nicht kritisch. Beispielsweise können die Lösungs- oder Verdünnungsmittel auch geringe Mengen an Wasser enthalten.

Bei der Hydrierung einer aromatischen Verbindung, in der mindestens eine Hydroxylgruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome, aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Bei der Hydrierung einer aromatischen Verbindung, in der mindestens eine Aminogruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie Ammoniak und Mono- oder Dialkylamine, in denen der Alkylrest vorzugsweise 1 bis 3 Kohlenstoffatome aufweist, wie z.B. Methyl-, Ethyl-, Propylamin oder die entsprechenden Dialkylamine.

Gemische dieser oder anderer Lösungs- oder Verdünnungsmittel können ebenfalls verwendet werden.

In beiden obigen Ausführungsformen ist die Menge des eingesetzten Lösungs- oder Verdünnungsmittels nicht in besonderer Weise beschränkt und kann je nach Bedarf frei gewählt werden, wobei jedoch solche Mengen bevorzugt sind, die zu einer 10 bis 80 Gew.-%igen Lösung der zur Hydrierung vorgesehenen Verbindung führen.

Besonders bevorzugt wird im Rahmen des erfindungsgemäßen Verfahrens das bei der Hydrierung dieses Verfahrens gebildete Produkt als Lösungsmittel eingesetzt, gegebenenfalls neben anderen Lösungs- oder Verdünnungsmitteln. In diesem Fall kann ein Teil des im Hydrierungsverfahren gebildeten Produktes den zu hydrierenden Verbindungen beigemischt werden. Bezogen auf das Gewicht der zur Hydrierung vorgesehenen aromatischen Verbindungen wird vorzugsweise die 1 bis 30-fache, besonders bevorzugt die 5 bis 20-fache, insbesondere die 5 bis 10-fache Menge des Hydrierungsproduktes als Lösungs- oder Verdünnungsmittel zugemischt.

Die Erfindung wird im folgenden anhand von einigen Ausführungsbeispielen näher erläutert.

### BEISPIELE

### Herstellung des Katalysators A (1 Gew.-% Ru/Al₂O₃)

14,2 g einer salpetersauren Rutheniumnitrosylnitratlösung (14 Gew.-% Ru) wurden mit 150 ml Wasser und 6 g eines hochmolekularen Natriumpolyacrylats (90% der Säuregruppen neutralisiert, vernetzt mit 0,4 mol-% Polyethylenglycol der Molmasse 1.500), welches das 300fache seines Eigengewichts an Wasser binden kann, versetzt. Nach 30 min wurde die gelartige Masse mit 280 g Al₂O₃ (Pseudoböhmit, BET-Oberfläche nach dem Calcinieren bei 600°C: 300 m²/g) verknetet. Nach Zugabe von 200 ml Ammoniaklösung (enthaltend 50 ml konz. Ammoniak) wurde 1 h lang geknetet. Die Masse wurde bei 6,5 · 10⁶ Pa (65 bar) in einer Strangpresse zu 3,8 mm-Strängen verformt, 16 h lang bei 120°C getrocknet und 6 h lang bei 30°C calciniert.

Der so hergestellte Katalysator wies folgende Analysedaten auf:

| | |
|---|---|
| Litergewicht | 425 g/l |
| | |
| BET-Oberfläche | 294 m²/g |
| | |
| Porenvolumen (DIN 66 132) | 0,81 ml/g |
| | |
| mittl. Durchmesser der Makroporen [nm] | 1.000 |
| | |
| mittl. Durchmesser der Mesoporen [nm] | 6 |
| | |
| Makroporenanteil [Vol.-%] | 35 |

Das Porenvolumen wurde gemäß DIN 66 132 bestimmt durch Quecksilberporosymmetrie gemäß der Anleitung von J. v. Brakel et al., *Powder Technology* 29 (1991) S. 1.

### Beispiel 1

1,2 l des oben beschriebenen Katalysators wurden in einen elektrisch beheizbaren Durchflußreaktor eingefüllt. Anschließend wurde ohne vorhergehende Aktivierung bei 2 · 10⁷ Pa (200 bar) und 160°C mit der Hydrierung von Anilin begonnen. Die Hydrierung wurde kontinuierlich in Sumpffahrweise ausgeführt, wobei ein Teil des Hydrierungsaustrags über eine Umlaufpumpe rückgeführt und dem Einsatzstoff vor dem Reaktor zugemischt wurde. Bezogen auf die Menge an Anilin wurde so die 10fache Menge an Hydrierungsprodukt als Lösungsmittel zugesetzt. Am Kopf des Abscheiders wurden 500 1 bis 600 l Wasserstoff/h entspannt. Die kontinuierlich dem Reaktor zugeführte Anilinmenge entsprach einer Katalysatorbelastung von 1,0 kg/l · h.

In Abhängigkeit von den Reaktionstemperaturen ergaben sich bei stationären Reaktionsbedingungen folgende Produktzusammensetzungen, wobei sich hier sowie in den folgen den Tabellen die Angabe "%" auf die Gaschromatographie-Flächen-% der den jeweiligen Produkten entsprechenden Peaks bezieht:

**Tabelle 1**

| Temperatur °C | CHA¹⁾ % | DCHA²⁾% | Anilin % | Cyclohexan + Cyclohexen % |
|---|---|---|---|---|
| 160 | 99,1 | 0,45 | 0,10 | 0,04 |
| 180 | 97,0 | 2,75 | 0,06 | 0,06 |
| 200 | 95,9 | 3,9 | - | 0,09 |

| | | | | |
|---|---|---|---|---|
| ¹⁾ CHA = Cyclohexylamin; | | | | |
| ²⁾ DCHA = Dicyclohexylamin | | | | |

### Beispiel 2

Es wurde eine Hydrierung gemäß Beispiel 1 durchgeführt, wobei jedoch zusätzlich kontinuierlich wasserfreier Ammoniak eindosiert wurde. Bezogen auf 100 Gew.-% Anilin wurden 10 Gewichtsteile Ammoniak zugesetzt. In Abhängigkeit von den Reaktionstemperaturen ergaben sich bei stationären Reaktionsbedingungen folgende Produktzusammensetzungen:

**Tabelle 2**

| Temperatur °C | CHA¹⁾ % | DCHA²⁾ % | Anilin % | Cyclohexan + Cyclohexen % |
|---|---|---|---|---|
| 180 | 99,3 | 0,08 | - | 0,07 |
| 200 | 98,4 | 0,8 | - | 0,09 |

| | | | | |
|---|---|---|---|---|
| ¹⁾ CHA = Cyclohexylamin; | | | | |
| ²⁾ DCHA = Dicyclohexylamin | | | | |

### Beispiel 3

In einem 3,5 l-Druckautoklaven wurden 2 kg einer Lösung von 50 Gew.-% Toluylendiamin (2,4-, 2,6-Diaminotoluol-Isomerengemisch) in Tetrahydrofuran und 500 ml des oben beschriebenen Katalysators eingeführt.

Anschließend wurde diskontinuierlich bei 150°C und 2 · 10⁷ Pa (200 bar) 5 h lang hydriert. Der Umsatz zum gewünschten cycloaliphatischen Diamin-Isomerengemisch war quantitativ, der Restaromatengehalt war kleiner als 0,01 Gew.-%.

### Beispiel 4

In einem 0,3 l Rührautoklav wurden 150 ml einer Lösung von 50% p-tert.-Butylphenol in Butanol und 15 ml des oben beschriebenen Katalysators gegeben.

Anschließend wurde bei 200 °C für 6 Stunden hydriert. Der Umsatz zum 4-tert.-Butylcyclohexanol war quantitativ, die Selektivität betrug 99,2 % bei einem cis/trans-Verhältnis von 48/52.

### Beispiel 5

In einem 0,3 l Rührautoklav wurden 150 ml einer Lösung von 50 % Bisphenol-A in Butanol und 15 ml des oben beschriebenen Katalysators gegeben.

Anschließend wurde bei 200 °C für 6 Stunden hydriert. Der Umsatz war quantitativ, die Selektivität zum 2,2-Bis-(4-hydroxycyclohexyl)-propan betrug 98,7 %.

## Patentansprüche

1. Verfahren zur Hydrierung einer aromatischen Verbindung, in der mindestens eine Hydroxylgruppe an einen aromatischen Kern gebunden ist, oder einer aromatischen Verbindung, in der mindestens eine Aminogruppe an einen aromatischen Kern gebunden ist, in Gegenwart eines Katalysators, der als katalytisch aktive Komponente Ruthenium alleine ode zusammen mit mindestens einem Metall der I., VII. oder VIII. Nebengruppe des Periodensystems, aufgebracht auf einem Träger, umfaßt, **dadurch gekennzeichnet, daß** der Katalysator erhältlich ist durch
a) Lösen der katalytisch aktiven Komponente oder einer Vorläuferverbindug davon in Wasser oder einem polaren, mit Wasser mischbaren Lösungsmittel,
b) Versetzen der so erhaltenen Lösung mit einem organischen Polymer, das in der Lage ist, mindestens das Zehnfache seines Eigengewichts an Wasser zu binden, wobei ein gequollenes Polymer erhalten wird
c) anschließendes Vermischen des gequollenen Polymers mit einem Katalysator-Trägermaterial, und
d) Formen der so erhaltenen Masse, Trocknen und Calcinieren,
wobei unter einer aromatischen Verbindung eine Verbindung verstanden wird, die eine Einheit der Struktur (I) aufweist: 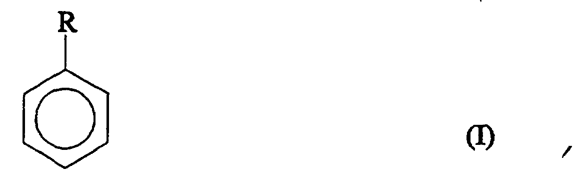 wobei R eine Hydroxyl- oder eine Aminogruppe dastellt.

2. Verfahren nach Anspruch 1, **dadurch gekennzeichnet, daß** die mindestens eine katalytisch aktive Komponente ausgewählt wird unter Ruthenium, Rhodium, Platin, Palladium, Kupfer, Rhenium, Cobalt, Nickel oder einem Gemisch aus zwei oder mehr davon.

3. Verfahren nach Anspruch 1 oder 2, **dadurch gekennzeichnet, daß** der Gehalt der katalytisch aktiven Komponente 0,01 bis 30 Gew,-%, bezogen auf das Gesamtgewicht des Katalysators, beträgt.

4. Verfahren nach einem der vorstehenden Ansprüche, **dadurch gekennzeichnet, daß** der Träger ein Aluminiumoxid, Siliciumdioxid, Kieselgur, ein Kieselgel, eine Tonerde, ein Silicat, ein Zeolith im Gemisch mit einem Aluminiumoxid, ein Zirconiumoxid, ein Titanoxid oder ein Gemisch aus zwei oder mehr davon ist.

5. Verfahren nach einem der vorstehenden Ansprüche, **dadurch gekennzeichnet, daß** eine aromatische Verbindung, in der mindestens eine Hydroxylgruppe an einen aromatischen Kern gebunden ist, wobei zusätzlich zur mindestens einen Hydroxylgruppe mindestens eine C₁₋₁₀-Alkylgruppe oder mindestens eine C₁₋₁₀-Alkoxygruppe oder mindestens eine C₁₋₁₀-Alkylgruppe und mindestens eine C₁₋₁₀-Alkoxygruppe, die wiederum jeweils substituiert sein können, an einen aromatischen Kern gebunden ist, oder eine aromatische Verbindung, in der mindestens eine Aminogruppe an einen aromatischen Kern gebunden ist, wobei zusätzlich zur mindestens einen Aminogruppe mindestens eine C₁₋₁₀-Alkylgruppe oder mindestens eine C₁₋₁₀-Alkoxygruppe oder mindestens eine C₁₋₁₀-Alkylgruppe und mindestens eine C₁₋₁₀-Alkoxygruppe, die wiederum jeweils substituiert sein können, an einen aromatischen Kern gebunden ist, hydriert wird.

6. Verfahren nach einem der vorstehenden Ansprüche, **dadurch gekennzeichnet, daß** die Hydrierung in Gegenwart eines Lösungs- oder Verdünnungsmittels durchgeführt wird.

## Claims

1. A process for hydrogenating an aromatic compound in which at least one hydroxyl group is bonded to an aromatic ring or an aromatic compound in which at least one amino group is bonded to an aromatic ring, in the presence of a catalyst comprising as catalytically active component either ruthenium alone or ruthenium together with at least one metal of transition group I, VII or VIII of the Periodic Table applied to a support, wherein the catalyst is obtainable by
a) dissolving the catalytically active component or a precursor compound thereof in water or a polar, water-miscible solvent,
b) admixing the solution thus obtained with an organic polymer which is able to bind at least ten times its own weight of water, giving a swollen polymer,
c) subsequently mixing the swollen polymer with a catalyst support material and
d) shaping, drying and calcining the composition obtained in this way, and the aromatic compound is a compound which contains of unit of the structure (I): 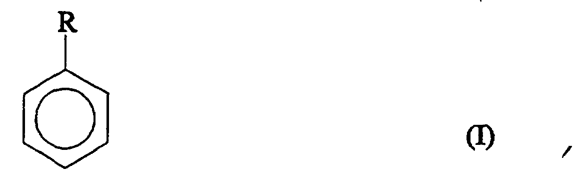
where R is a hydroxyl or amino group.

2. A process as claimed in claim 1, wherein the catalytically active component or components is/are selected from the group consisting of ruthenium, rhodium, platinum, palladium, copper, rhenium, cobalt, nickel and mixtures of two or more thereof.

3. A process as claimed in claim 1 or 2, wherein the content of the catalytically active components is from 0.01 to 30% by weight, based on the total weight of the catalyst.

4. A process as claimed in any of the preceding claims, wherein the support is an aluminum oxide, silicon dioxide, kieselguhr, a silica gel, an alumina, a silicate, a zeolite in admixture with an aluminum oxide, a zirconium oxide, a titanium oxide or a mixture of two or more thereof.

5. A process as claimed in any of the preceding claims, wherein an aromatic compound in which at least one hydroxyl group is bonded to an aromatic ring and, in addition to the hydroxyl group or groups, at least one C₁-C₁₀-alkyl group or at least one C₁-C₁₀-alkoxy group or at least one C₁-C₁₀-alkyl group and at least one C₁-C₁₀-alkoxy group, each of which may in turn be substituted, is/are bonded to an aromatic ring, or an aromatic compound in which at least one amino group is bonded to an aromatic ring and, in addition to the amino group or groups, at least one C₁-C₁₀-alkyl group or at least one C₁-C₁₀-alkoxy group or at least one C₁-C₁₀-alkyl group and at least one C₁-C₁₀-alkoxy group, each of which may in turn be substituted, is/are bonded to an aromatic ring, is hydrogenated.

6. A process as claimed in any of the preceding claims, wherein the hydrogenation is carried out in the presence of a solvent or diluent.

## Revendications

1. Procédé d'hydrogénation d'un composé aromatique, dans lequel au moins un radical hydroxyle est relié sur un noyau aromatique, ou un composé aromatique, dans lequel au moins un radical amino est relié sur un noyau aromatique, en présence d'un catalyseur, qui comprend comme composant catalytiquement actif, le ruthénium, seul ou avec au moins un métal des groupes secondaires I, VII ou VIII du système périodique, appliqués sur un support, **caractérisé en ce que** le catalyseur est accessible par :
a) dissolution du composant catalytiquement actif ou d'un composé précurseur de celui-ci dans de l'eau ou un solvant polaire, miscible à l'eau ;
b) addition à la solution ainsi obtenue, d'un polymère organique, qui est en mesure de lier l'eau à au moins 10 fois son propre poids, où on obtient un polymère gonflé ;
c) ensuite, mélange du polymère gonflé avec un matériau de support pour catalyseur, et
d) moulage de la masse ainsi obtenue, séchage et calcination, où par composé aromatique, on entend un composé qui présente une unité de la structure (I) : 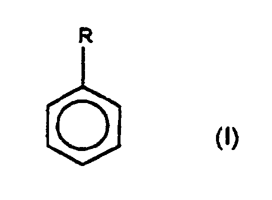
où R représente un radical hydroxyle ou amino.

2. Procédé selon la revendication 1, **caractérisé en ce que** le au moins un composant catalytiquement actif est choisi parmi le ruthénium, le rhodium, le platine, le palladium, le cuivre, le rhénium, le cobalt, le nickel ou un mélange de deux ou plusieurs de ceux-ci.

3. Procédé selon la revendication 1 ou 2, **caractérisé en ce que** la teneur en le composant catalytiquement actif se situe dans l'intervalle allant de 0,01 à 30% en poids, sur base du poids total du catalyseur.

4. Procédé selon l'une quelconque des revendications précédentes, **caractérisé en ce que** le support est un oxyde d'aluminium, un oxyde de silicium, la terre d'infusoires, un gel de silice, une alumine, un silicate, une zéolite en mélange avec un oxyde d'aluminium, un oxyde de zirconium, un oxyde de titane ou un mélange de deux ou plusieurs de ceux-ci.

5. Procédé selon l'une quelconque des revendications précédentes, **caractérisé en ce que** l'on réalise l'hydrogénation d'un composé aromatique, dans lequel au moins un radical hydroxyle est lié sur un noyau aromatique, où en plus du au moins un radical hydroxyle, est lié sur un noyau aromatique, au moins un radical alkyle en C₁-₁₀ ou au moins un radical alcoxy en C₁₋₁₀, ou au moins un radical alkyle en C₁₋₁₀ et au moins un radical alcoxy en C₁₋₁₀, qui peuvent à nouveau être chaque fois substitués, ou d'un composé aromatique, dans lequel au moins un radical amino est lié sur un noyau aromatique, où en plus du au moins un radical amino, est lié sur un noyau aromatique, au moins un radical alkyle en C₁₋₁₀ ou au moins un radical alcoxy en C₁₋₁₀, ou au moins un radical alkyle en C₁₋₁₀ et au moins un radical alcoxy en C₁₋₁₀, qui peuvent à nouveau être chaque fois substitués.

6. Procédé selon l'une quelconque des revendications précédentes, **caractérisé en ce que** l'on réalise l'hydrogénation en présence d'un solvant ou agent de dilution.
